# EUROPÄISCHE PATENTSCHRIFT

(11) **EP 0 892 653 B1**
(45) Veröffentlichungstag und Bekanntmachung des Hinweises auf die Patenterteilung: **02.07.2003**
(21) Anmeldenummer: 97919275.4
(22) Anmeldetag: 03.03.1997
(51) Int. Cl.: A61N 1/05

(54) **ELEKTRODENANORDNUNG**
ELECTRODE ARRANGEMENT
SYSTEME D'ELECTRODES

(30) Priorität: 01.03.1996 DE 19609471
(43) Veröffentlichungstag der Anmeldung: 27.01.1999
(73) Patentinhaber: BIOTRONIK Mess- und Therapiegeräte GmbH & Co Ingenieurbüro Berlin, 12359 Berlin (DE)
(72) Erfinder: HARTUNG, Wolfgang, D-39114 Magdeburg (DE)
(74) Vertreter: Christiansen, Henning, Dipl.-Ing.
(86) Internationale Anmeldenummer: DE9700520
(87) Internationale Veröffentlichungsnummer: WO97031678

(56) Entgegenhaltungen:
- EP-A- 0 601 338
- US-A- 3 903 897
- US-A- 4 825 871
- US-A- 5 172 694

## Beschreibung

Die Erfindung betrifft eine Elektrodenanordnung der im Oberbegriff des Anspruchs 1 angegebenen Art.

Bei der Therapie verschiedener chronischer Herzrhythmusstörungen sind implantierte Herzschrittmacher in Verbindung mit auf einem intrakardialen Elektrodenkatheter angeordneten und an der Herzinnenwand positionierten Reizelektroden in Gebrauch, über die das reizbare Herzgewebe erregt wird. Die Ausgestaltung der Schrittmacher und der zugehörigen Elektrodenleitungen hat zunehmende Vervollkommnung erfahren, wobei zahlreiche technische Lösungen zur Verankerung der Elektrodenkatheter an der Herzwand - sowohl in der Herzkammer (Ventrikel) als auch im Vorhof (Atrium) - gefunden wurden und tatsächlich wesentliche praktische Verbesserungen gelungen sind.

Dennoch zählen auch heute noch Elektrodendislokationen, die zu einer Verschlechterung oder gar einem Verlust des Kontakts mit dem reizbaren Gewebe an der Herzinnenwand und in der Folge zumindest zu einer wesentlichen Erhöhung der Reizschwelle und damit einem erhöhten Stromverbrauch des Schrittmachers und einer verringerten Lebensdauer und schlimmstenfalls zu dessen Funktionsunfähigkeit führen, zu den wichtigsten Komplikationen bei der Schrittmachertherapie.

Weiterhin kann der Umstand, daß bis dato die Reizimpulse eines implantierten Schrittmachers über wandständige Elektroden an das reizbare Muskelgewebe der Herzinnenwanding im unteren Bereich des Ventrikels bzw. des Atriums übertragen werden, auch aus grundsätzlichen physiologischen Erwägungen heraus nicht befriedigen.

Flottierende Elektroden konnten andererseits bisher nicht mit so großer Sicherheit die Stimulation sicherstellen, dass eine regelmäßige Anwendung vertretbar gewesen wäre.

Die US 4,825,871 betrifft eine Anordnung zur Behandlung tachyarrythmischer Phänomene. Die Anordnung umfasst eine Elektrodenleitung mit zumindest einer Elektrode, die in das rechte Atrium des Herzens eingeführt wird. In Abhängigkeit vom Signal eines Herzschlagsensors wird die Abgabe eines Impulses an das Herzgewebe gesteuert.

In der US 5.172,694 wird eine Elektrodenleitung mit zumindest zwei flotierenden Bipolen im Bereich des Atriums des Herzens beschrieben.

Der Erfindung liegt daher die Aufgabe zugrunde, eine verbesserte Elektrodenanordnung der eingangs genannten Gattung anzugeben, die einen hinsichtlich der Reizimpulslokalisierung besser den physiologischen Gegebenheiten folgenden und zuverlässigeren Betrieb eines Herzschrittmachers ermöglicht.

Diese Aufgabe wird durch eine Elektrodenanordnung mit den Merkmalen des Anspruchs 1 gelöst.

Die Erfindung schließt den Gedanken ein, eine Elektrodenanordnung und -ansteuerung zu realisieren, mit der die Reizimpulse des Schrittmachers den in der Reizbildungshierarchie hochstehenden Bereichen des kardialen Reizbildungs- und -leitungssystems - primär dem Sinusknoten und sekundär dem AV-Knoten - zugeführt werden können, um etwa ein Sinusknotensyndrom direkt am Entstehungsort zu therapieren oder im Falle eines AV-Blocks eine Stimulation unmittelbar an dem vom physiologischen Defekt primär betroffenen Bereich vornehmen zu können.

Zur praktischen Umsetzung dieser Gedanken bedient sich die vorgeschlagene Elektrodenanordnung des Mittels einer "Fokussierung" des bei gleichzeitiger Abgabe von Reizimpulses mit entgegengesetzter Polarität in der Umgebung zweier oder mehrerer Elektroden In geeigneter geometrischer Elektrodenanordnung ausgebildeten elektrischen Feldes auf den Sinusknoten bzw. den AV-Knoten oder den sonstigen zu stimulierenden Bereich.

Auf diese Weise ist es möglich, auch bei flottierender Elektrode eine so starke elektrische Reizwirkung lokal zu konzentrieren, dass ein körperlicher Kontakt zwischen Gewebe und Reizelektrode nicht erforderlich ist.

Des weiteren ist für die Erfindung von besonderer Bedeutung, dass das Dipolfeld im Bereich des Gewebes verzerrt ist. Das Schrittmachergehäuse als indifferente Elektrode bewirkt eine starke Verzerrung des Dipolfeldes. Begünstigt wird dieser Effekt durch die Unstetigkeit des Potentiallinienverlaufs im Übergangsbereich Gewebe Blut wegen des im Blut geringeren spezifischen Widerstands.

Die Erhöhung des Potentialgradienten durch die geeignete geometrische Elektrodenanordnung einerseits und die entgegengesetzte Polarität der Impulse andererseits ermöglicht die Überschreitung der Reizschwelle in zu den Elektroden nicht direkt benachbarten Gewebebereichen und damit die Reizung der nicht an der Oberfläche der Herzwand und zudem in Gebieten des Herzens, in denen eine Fixierung von Elektroden praktisch unmöglich ist, liegenden Zielbereiche für die Erregung, nämlich des Sinus- bzw. AV-Knotens.

In einer hinsichtlich der Steuerung des Erregungs-Zielbereiches besonders vorteilhaften Ausgestaltung der Elektrodenanordnung umfaßt diese in räumlicher Beziehung zur ersten und zweiten Elektrode mindestens eine dritte Elektrode, die derart ausgebildet und angeordnet und mit einer vorbestimmten dritten Spannung beaufschlagt ist, dass sie den Potentialgradienten im Bereich des Sinusknotens oder des AV-Knotens über den allein mit der ersten und zweiten Elektrode (bei als vorgegeben vorausgesetzter Spannungsamplitude) erreichbaren Wert hinaus erhöht.

In einer in vorteilhafter Weise auf herkömmliche Weise zu konstruierenden und günstig zu implantierenden Ausführung sind auch die zweite und/oder weitere Elektrode(n) auf der intrakardial verlegten Elektrodenleitung angeordnet.

Diese weist zweckmäßigerweise eine derart bemessene Länge und/oder Krümmung auf, dass die Elektroden nach Einführung in das Herz im Bereich des Atriumdaches nahe der Einmündung der Vena cava superior bzw. nahe des Septums und der Einmündung des Sinus coronarius in das Atrium , d.h. - je nachdem, ob der Sinusknoten oder der AV-Knoten erregt werden soll - in geringer Entfernung hierzu, angeordnet sind.

Dabei ist in einer bevorzugten Ausgestaltung die Elektrodenleitung derart ausgebildet, daß die dritte und/oder weitere Elektrode(n) oberhalb der ersten und zweiten Elektrode, insbesondere annähernd auf deren verlängerter gerader Verbindungslinie - für den Fall der Reizung des Sinusknotens insbesondere in der vena cava superior - angeordnet sind.

Ist hierbei gemäß der oben erwähnten Ausgestaltung eine dritte Elektrode vorgesehen, so ist diese im wesentlichen am Scheitelpunkt der Krümmung der Elektrodenleitung angeordnet derart, dass sie einen Abstand zu einer geraden Verbindungslinie zwischen der ersten und zweiten Elektrode hat.

Da eine Fixierung der Elektrodenleitung an der Herzwand bei der erfindungsgemäßen Elektrodenanordnung nicht erforderlich ist, kann diese im Herzen "schwimmen". Sie wird mithin zweckmäßigerweise mit einer solchen Biegesteifigkeit aufgebaut und gegebenenfalls einer solchen vorgeprägten Krümmung im Längsverlauf versehen, dass die Elektroden nach Einführung in das Herz im wesentlichen nicht in direktem Kontakt mit der Herzwandung stehen, aber nahe dem zu reizenden Bereich angeordnet sind.

In einer bevorzugten Ausführung weist die Elektrodenleitung eine - in einer besonderes variablen Ausführung auch nach dem Einführen noch verschiebbare - Verzweigung auf, und die zweite oder eine weitere Elektrode ist auf einem von der Hauptleitung abzweigenden Teil der Elektrodenleitung angeordnet, ist, während mindestens zwei Elektroden (darunter die erste) auf der Hauptleitung angeordnet sind.

Mit dieser Ausführung ist ein ähnliches Ergebnis erreichbar wie mit dem Vorsehen einer Krümmung und einer dritten Elektrode im Bereich des Scheitelpunktes der Krümmung, nämlich eine Potentialliniendeformation in Richtung auf den Zielbereich hin. Mit Hilfe einer Abzweigung kann diese Verschiebung besser räumlich gesteuert werden als mit einer einsträngigen Elektrodenleitung.

Die Krümmung bzw. das Abstehen des von der Hauptleitung abzweigenden Teiles der Elektrodenleitung werden durch geeignete (als solche bekannte) Mittel nach der Einführung erzeugt, bevorzugt etwa durch Elemente aus einer bei Körpertemperatur aktivierten Gedächtnislegierung oder Vorspannelemente umfassen, die bei Entfernung eines zum Einführen dienenden Führungsdrahtes aus der Elektrodenleitung eine vorgeprägte Form annehmen. Es ist auch möglich, Mittel zur Veränderung der Krümmung von außen vorzusehen.

Eine gegenüber Elektrodenleitungen mit zur Mittelachse symmetrischer Elektrodenkonfiguration verbesserte Möglichkeit der Annäherung der Elektroden an den zu beeinflussenden Gewebsbereich bietet eine Ausführung, bei der die Elektroden bezüglich einer Mittelebene der Elektrodenleitung asymmetrisch auf dieser angeordnet sind. Dabei kann ein Teil der Elektroden auf einer Seite der Leitung und ein anderer Teil auf der gegenüberliegenden angeordnet sein, es kann aber auch zweckmäßig sein, alle Elektroden auf derselben Seite der Leitung anzubringen.

In einer weiteren bevorzugten Ausführung ist die dritte (oder eine weitere) Elektrode als eine außerhalb des Herzens angeordnete Flächenelektrode, insbesondere als die Spannungsquelle und die Herzschrittmacher aufnehmendes Schrittmachergehäuse, ausgebildet, wobei diese Flächenelektrode insbesondere als indifferente Elektrode geschaltet, d.h. mit Nullpotential verbunden, ist.

Die Elektrodenleitung kann bevorzugt mindestens eine Gruppe von vier in axialer Richtung gegeneinander beabstandeten Elektrodenabschnitten aufweisen, wobei der erste Spannungsimpuls an den ersten und dritten oder zweiten und vierten Elektrodenabschnitt der Gruppe angelegt wird, so daß sich ein erstes elektrisches Dipolfeld zwischen diesen Abschnitten aufbaut, und der zweite Impuls an die jeweils verbleibenden Elektrodenabschnitte der Gruppe angelegt wird, so daß sich ein gegenüber dem ersten Dipolfeld räumlich versetztes zweites Dipolfeld zwischen diesen Elektrodenabschnitten aufbaut.

Insbersondere sind die Elektroden unterschiedlicher Polarität derart auf der Elektrodenleitung angeordnet und/oder das Verhältnis der Potentiale ist derart gewählt, daß die sich einstellenden Äuipotentiallinien (und damit auch die im wesentlichen die Feldlinien) am Stimulationsort in die Herzwand ein und aus dieser wieder austreten.

Für diese oder ähnliche Ausgestaltungen, bei denen mehrere Elektrodengruppen impulsartig anzusteuern sind, sind zweckmäßigerweise zwei (oder mehrere) Impulserzeuger in dem Herzschrittmacher vorgesehen, die jeweils im wesentlichen zeitgleich mindestens einen ersten und einen zweiten Spannungsimpuls mit entgegengesetzter Polarität erzeugen können.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 eine schematische Darstellung des Reizbildungs- und Erregungsleitungssystems des Herzens,
Figuren 2 und 2a bis 2d schematische Darstellungen des Feldlinienverlaufes bei verschiedenen Elektrodenanordnungen zur Erläuterung eines Grundgedankens der Erfindung einschließlich Aktivierungsfunktionen,
Figur 3 eine weitere schematische Darstellung des Verlaufs des elektrischen Felds bei einer weiteren Ausführung der Erfindung sowie
Figuren 4a und 4b schematische Darstellungen von Elektrodenanordnungen gemäß einer weiteren Ausführungsform der Erfindung (in zwei Varianten).

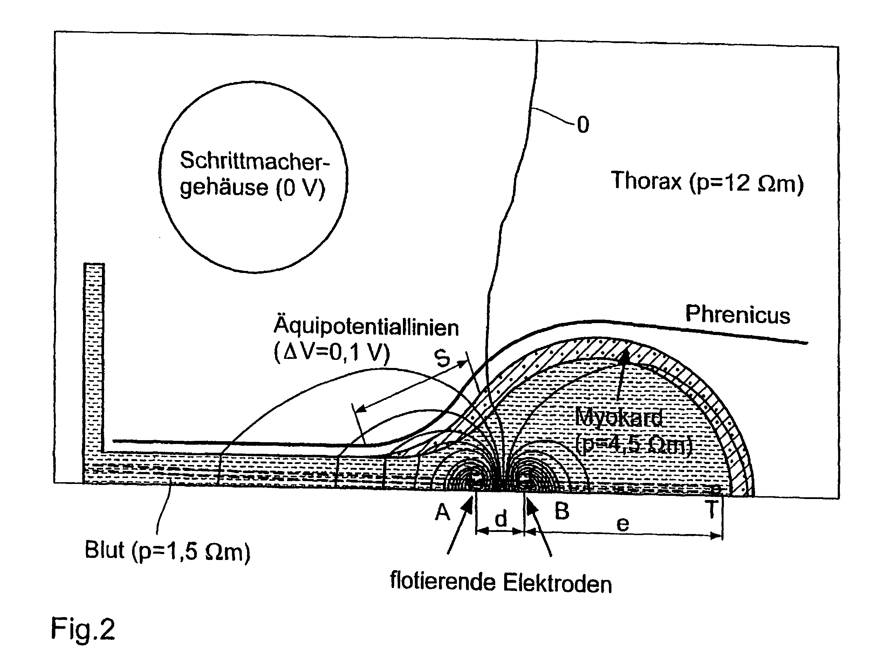

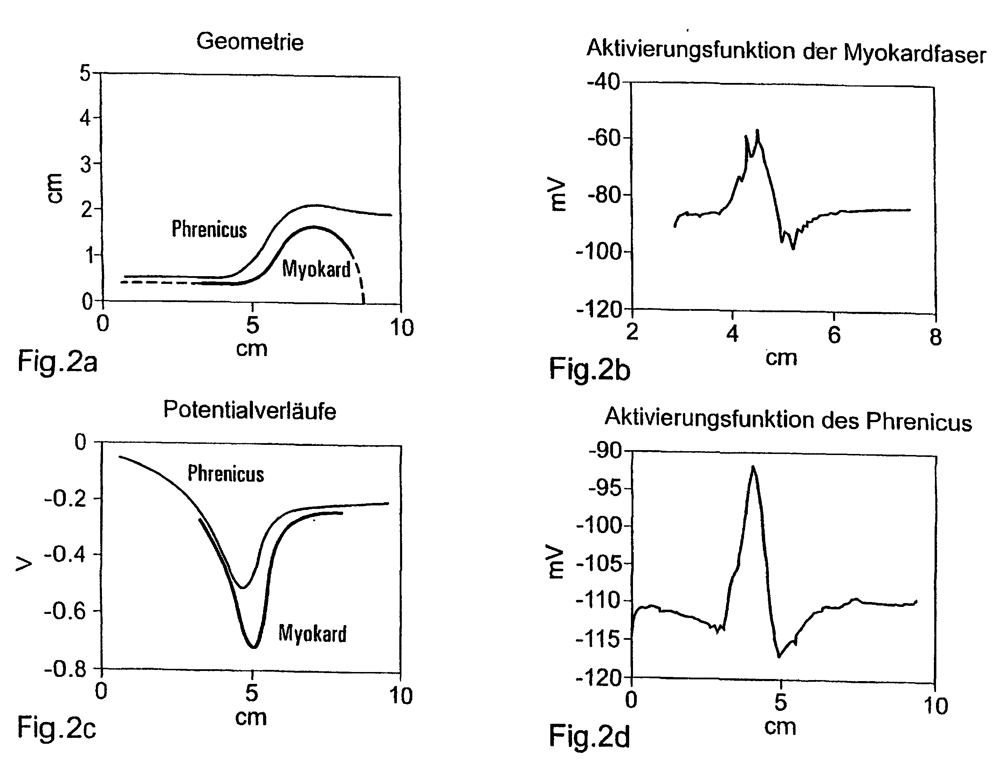

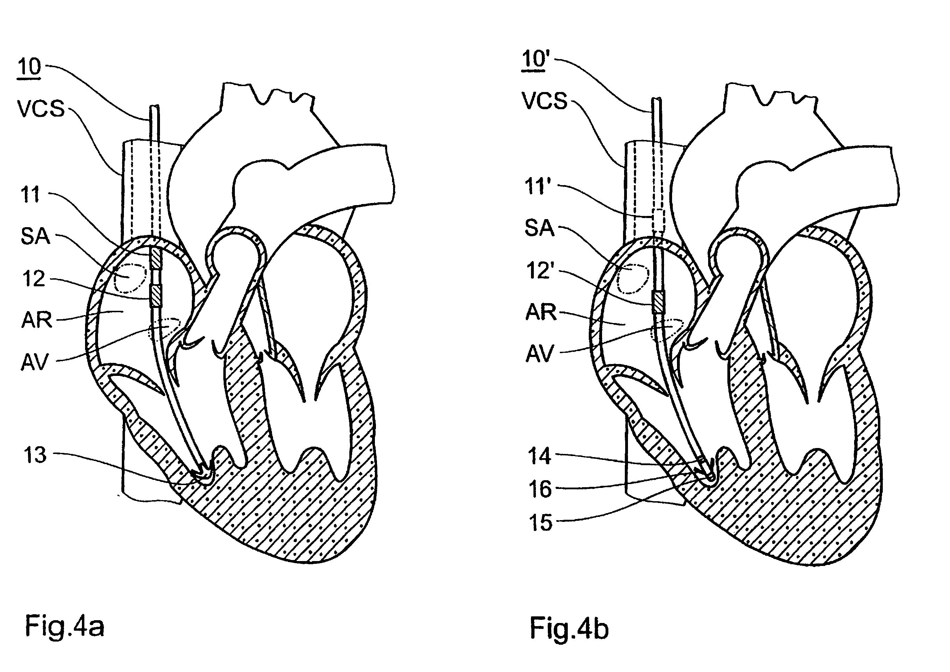

Die Figuren 2 und 4 fallen nicht unter Anspruch 1, weil die Elektrodenleitungen keine Verzweigung aufweisen. Diese Figuren sind jedoch nützlich, um eine mögliche elektrische Feldverteilung zu erläutern.

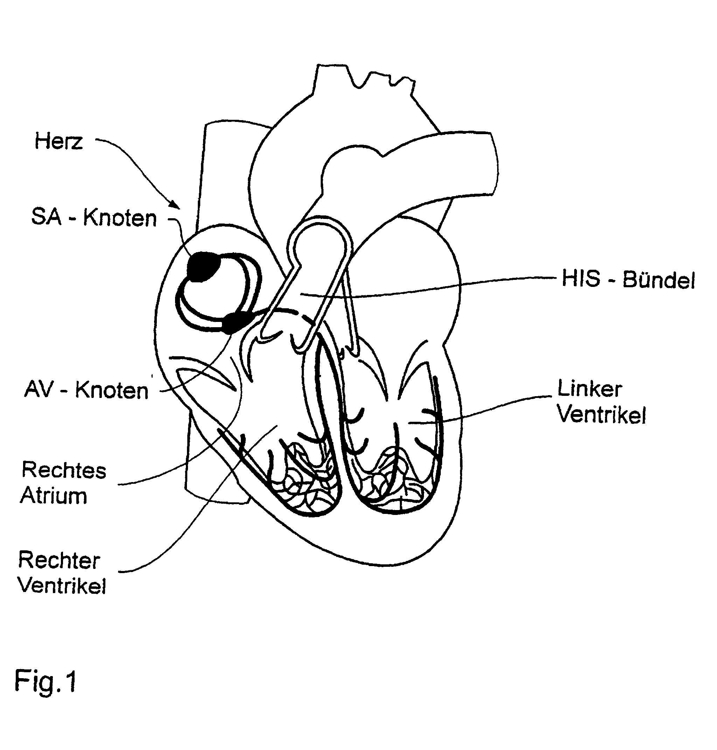

Die Lage der Elemente des Reizbildungs- und Erregungsleitungssystems im Herzen (wozu auch das oben nicht erwähnte His-Bündel und der - jeweils in die Purkinje-Fasern mündende - linke bzw. rechte Schenkel ("Left" bzw. "Right Bundle Branch") gehören - ist zunächst in Fig. 1 skizzenartig gezeigt.

Figur 2 zeigt in schematischer Darstellung den Feldlinienverlauf bei einer Elektrodenanordnung zur Erläuterung eines Grundgedankens der Erfindung. Die dargestellten Feldlinien sollen nicht maßstäblich die Feldstärke abbilden, sondern sind so gewählt, daß bestimmte Verhältnisse in der FeldGeometrie deutlich werden.

In der schematischen Darstellung ist die Blut-/Gewebe-Verteilung im Atrium und der angrenzenden "Vena cava superior" dargestellt. Weiterhin wiedergegeben ist der Myokard des Atriums und der Phrenicus-Nerv und der umgebende Thorax. Eine dritte Elektrode - hier das Schrittmachergehäuse - befindet sich auch im Stimulationsfall auf dem Potential 0 V. Die flottierende (gestrichelt dargestellte) Elektrodenleitung trägt die Elektroden A und B, welche einen elektrischen Dipol bilden, wobei im vorliegenden Beispiel die Elektrode A negativ (-1,5 V) und die Elektrode B (+1,5 V) positiv gegenüber dem Schrittmachergehäuse gepolt ist. Es ist ersichtlich, dass bei der dargestellten Elektrodenposition die Potentiallinie von 0 Volt (in der Zeichnung nahezu senkrecht nach oben gerichtet) eine Verformung zur dritten Elektrode (Schrittmachergehäuse) hin bewirkt.

Die spezifischen Widerstände sind ebenfalls in Figur 2 angegeben. Es ist ferner ersichtlich, dass bei Betrachtung des Schrittmachergehäuses als idealen Leiter (äquipotential bei 0 V) und bei der dargestellten Elektrodenposition sich eine Verzerrung des Dipolfeldes im Bereich des Myokards ergibt.

Dies wird erreicht durch die in Figur 2 wiedergegebene Elektrodengeometrie, welche bestimmt wird durch den Abstand d zwischen den Elektroden und den Abstand e von den Elektroden bis zum Ende der Elektrode, die in der Herzspitze gelegen ist und beim dargestellten Ausführungsbeispiel eine konventionelle (unipolare) Ventrikelelektrode T trägt. Der Abstand e ist im dargestellten Beispiel auf die Mitte des Abstands d der beiden Elektroden A und B bezogen. Je nach Größe des menschlichen Herzens kann die Größe e variieren, wobei in vielen Fällen bereits durch eine geeignete Positionierung ein Maximum der Stimulationseffektivität erzielt werden kann. Der Abstand d wird so gewählt, dass ein Bereich maximaler Krümmung den Stimulationsbereich S durchdringt. Hierbei verringert ein größerer Abstand die Krümmung. Ein zu kleiner Abstand lässt hingegen den Feldbereich maximal gekrümmter Äquipotentiallinien den Stimulationsbereich nicht erreichen. Insoweit kann hier gegebenenfalls durch Versuche ein optimaler Stimulationseffekt erreicht werden.

Eine weitere Variable im Bild ist die Symmetrie der Spannung an den Elektroden A und B. Durch eine Vergrößerung des Potentials der Elektrode B läßt sich die indifferente Potentiallinie 0 V ebenfalls in den zu stimulierenden Bereich in verschieben. Dieser Effekt hat jedoch im Vergleich zu den geometrischen Größen einen relativ geringeren Einfluß, ist jedoch in Bezug auf das Verhältnis der Stimulation von Myocardfaser und Phrenicus von Bedeutung, wie weiter unten näher ausgeführt werden wird.

Mit der im Bild schematisch dargestellten Elektrodengeometrie läßt sich die beschriebene Potentialverteilung im Eingangsbereich der Vena Cava Superior zum Atrium hin gut erreichen. In Figur 2 ist ersichtlich, daß im Bereich S die Krümmung der Äquipotentiallininen so groß ist, daß auch die senkrecht dazu verlaufenden Feldlinien im Stimulationsbereich in die Herzwand ein und wieder austreten. Damit zeichnet sich der Stimulationsbereich durch eine Zone aus, in der die erzeugten Feldstärken ein Maximum sind.

In den Figuren 2a bis d ist der Einfluß dargestellt, der sich durch die Impulssymmetrie auf die Stimulation von Herzmuskelzellen und Phrenicus ergibt. In Figur 2a in einer vereinfachten Modellgeometrie der relative Verlauf von Phrenicus und Myokard wiedergegeben. Der Phrenicus ist als durchgehenden Nervenstrang aufzufassen und seine Erregbarkeit hat einen charakteristischen Verlauf, der weiter unten näher dargestellt ist.

Die Aktivierungsfunktion der Mycardfaser ist in Figur 2b) näher dargestellt. Sie liegt bei - 90 mV. Als Myocardbestandteil wird eine Muskelfaser betrachtet, die sich von der Einmündung der vena cava superior ins Atrium hinein bis zur Ventilebene erstreckt. Das Ruhemembranpotential und die Reizschwelle liegen bei -90 mV bzw. bei - 60 mV. Die Stimulation der Myocardfaser erfolgt überschwellig.

Es stellt sich bei der Schrittmacherstimulation des Herzens das Problem, dass eine Erregung des Phrenicus vermieden werden soll, da seine Stimulation zu unerwünschtem Zucken des Diaphragmas oder der Schrittmachertasche führt.

Im Gegensatz zu Herzmuskelzellen haben Nervenzellen ein Ruhemembranpotential von etwa -110 mV. Die Reizschwelle liegt um -90 mV und ist damit unterschwellig.

Um dafür Sorge zu tragen, dass die Myocardfasem oberschwellig, die Nervenzellen aber nicht stimuliert werden, wird bei der hier dargestellten Schrittmacherelektrode gleichzeitig der "Störabstand" zwischen gewünschter Myocardstimulation und unerwünschter Phrenicusstimulation optimiert. Dies wird erreicht durch die gewählte Unssymmetrie der Elektrodenpotentiale A und B. Ausgehend von einer Gesamtspannung von 3 V und dem Ziel, das Myokard einerseits möglichst sicher zu stimulieren und andererseits gleichzeitig die Phrenicusstimulation möglichst zu vermeiden bzw. zu minimieren ergibt sich das in Fig. 2b dargestellte Bild der Potentialverläufe für eine Impulsspannung von -2,55 V der Elektrode A und einer Impulsspannung von + 0,45V der Elektrode B. Der Unsymmetriefaktor beträgt in diesem (Modell-) Fall 1,7/0,3. Hierbei werden Rechteckimpulse zugrundegelegt, welche konphasisch, also zeitsynchron ohne Versatz abgegeben werden.

Dieses Optimum ist von Patient zu Patient unterschiedlich und kann durch individuelle Programmierung erfolgen, nachdem die Elektrode im Herzen verlegt ist. Hierbei ist zu berücksichtigen, daß Myokard und Phrenicus nicht in einer Ebene gelegen sind. Der sich tatsächlich einstellende Störabstand ist also weitaus besser als der hier an einem zweidimensionalen Modell errechnete theoretische Wert. Es ist zu berücksichtigen, daß gemäß der Erfindung eine Fokussierung zunächst auf die Myocard-Stimulation erfolgen muß. Durch eine Veränderung der Symmetrie der Impulse kann dann nachträglich versucht werden, eine eventuell zu bemerkende störende Stimulation des Phrenicus durch Umprogrammierung der Symmetrie und somit "Ausblenden" zu beseitigen.

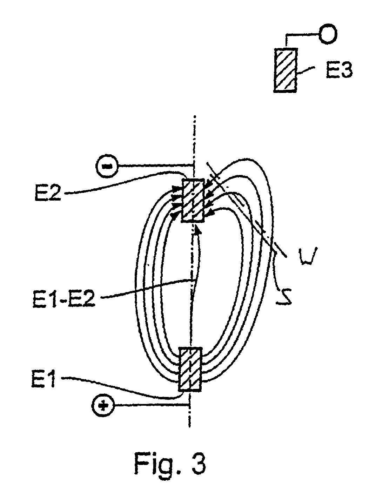

In Fig. 3 ist das elektrische Feld gezeigt, das sich zwischen zwei mit dem positiven Pol "+" bzw. dem negativen Pol "-" einer Spannungsquelle verbundenen Elektroden E1 und E2 mit gegenüber ihrem Abstand kleinen Abmessungen (im freien Raum) ausbildet. Es entspricht näherungsweise dem Feld zwischen zwei Punktladungen mit unterschiedlichem Vorzeichen. Der Potentialgradient ist auf der geraden Verbindungslinie E1-E2 zwischen den beiden Elektroden E1. E2 am größten, und die Feldstärke und damit auch der Potentialgradient nimmt in erster Näherung entsprechend dem Coulombschen Gesetz mit wachsendem Abstand von dieser Linie ab. Anstelle der Äquipotentiallinien sind hier die verformten elektrischen Feldlinien dargestellt, stets senkrecht zu den Äquipotentiallinien gerichtet sind.

Wie ersichtlich ist, tritt ein ähnlicher, aber hinsichtlich der Verbindungslinie E1-E2 zwischen der ersten und zweiten Elektrode E1, E2 asymmetrischer, Effekt bei Plazierung einer dritten Elektrode E3" mit einem Abstand von der verlängerten Verbindungslinie E1-E2 auf. Im der dritten Elektrode abgewandten Halbraum hat sich - wiederum gleiche Spannungen wie bei den übrigen Figuren vorausgesetzt - der Wirkungsbereich des Feldes verkleinert, in dem ihr zugewandten Halbraum hat er sich ausgeweitet. Es ist ersichtlich, daß in dem Bereich der Elektrode E2, die der Elektrode E3 zugewandt ist, eine starke Verzerrung der Feldlinien - in Richtung auf E3 zu - erfolgt ist. Folgt die Herzwand einem in Figur 3 dargestellten und mit W bezeichneten gestrichelten Verlauf, so treten die Feldlinien im Stimulationsbereich S in diese Herzwand ein und auch wieder daraus hervor.

Ähnliche, für den Fachmann aufgrund der Gesetze der Elektrostatik berechenbare Effekte treten bei veränderten Elektrodenpolaritäten und räumlichen Elektrodenanordnungen auf, wobei die konkreten Feldlinienbilder natürlich auch von den Beträgen der angelegten Spannungen abhängen.

Die oben skizzierten und erläuterten Verhältnisse gelten - wie Untersuchungen bestätigt haben - näherungsweise auch für (als solche bekannte) Elektrodenleitungen bzw. - katheter mit einer isolierenden Umhüllung und mehreren voneinander beabstandeten und isolierten Elektrodenabschnitten und insbesondere auch für Elektrodenanordnungen in einer physiologischen Flüssigkeit.

Aufgrund der Möglichkeit, Elektrodenkatheter im Herzen im wesentlichen geradlinig flottierend zu verlegen, kann eine Vielzahl von Konfigurationen angegeben werden, mit denen die Erfindung realisiert und über ein in ihrer Umgebung erzeugtes, hinreichend starkes elektrisches Feld direkt der Sinus- oder der AV-Knoten erregt wird.

Nachfolgend werden hierfür Beispiele angegeben, die jedoch nicht im Sinne einer Beschränkung zu verstehen sind.

Die Figuren 4a und 4b sind schematische Darstellungen zweier mit geradlinigem Verlauf über die Vena cava superior VCS schwimmend in das rechte Atrium AR eines Herzens H verlegte Elektrodenleitungen 10 bzw. 10', auf denen jeweils zwei Elektroden(abschnitte) 11 und 12 bzw. 11' und 12' gebildet sind. Beide Leitungen sind in der Länge und im Abstand der Elektroden 12 und 13 bzw. 12' und 13' so ausgebildet und so weit in das Atrium hinein verlegt, daß sie im bipolaren Betrieb, d.h. bei Anlegen einer Spannung mit unterschiedlicher Polarität, den Sinusknoten SA erregen können, ohne wandständig fixiert oder auch nur positioniert sein zu müssen. Die in beiden Figuren dargestellten Elektroden sind für Zweikammerbetrieb geeignet. In Figur 4a ist eine herkömmliche Ventrikelelektrode 13 mit Fixationsmitteln am Ende der Elektrodenleitung vorgesehen. In Figur 4b ist eines entsprechende Elektrode vorgesehen, welche an ihrem freien Ende im Ventrikel ein ebenfalls flottierendes Elektrodenpaar 14 und 15 aufweist. Diese Elektroden werden nach entsprechenden geometrischen Grundsätzen wie die Atriumelektroden dimensioniert und sind auf das Endocard des Ventrikels gerichtet, ohne dort fest verankert zu sein. In ihrem Endbereich weisen sie dünne Tines 16 aus elastischem Material auf, welche in Bezug auf das Elektrodenende zurückgesetzt sind und nicht die Aufgabe haben, das Elektrodenende mit den Stimulationselektroden in der Nachbarschaft des Gewebes zu Fixieren, sondern sollen nur dazu dienen, das Elektrodenende etwa im mittleren Querschnitt der Kammer in der Nähe ihres unteren Endes zentriert zu halten.

Es ist ersichtlich, da der erfinderische Effekt auch mit mehreren Elektroden erreicht werden kann. So können zwei Elektroden im Atrium angeordnet oder aber auch eine weitere Elektrode in der Vena cava superior gelegen sein. Der Einsatz der einen oder der anderen Elektrodenanordnung kann von der tatsächlichen Lage des Sinusknotens bei einem Patienten und von dessen Reizschwelle abhängen.

## Patentansprüche

1. Elektrodenanordnung zur Stimulation des Herzens mittel eines implantierbaren Herzschrittmachers zur Erzeugung von elektrischen Impulsen, mit wenigstens einer mit einem ersten Ausgang des Herzschrittmachers verbindbaren, auf einer intrakardial verlegbaren nichtwandständigen Elektrodenleitung angeordneten ersten Elektrode (E1) und einer von der ersten Elektrode beabstandeten und mit einem zweiten Ausgang des Herzschrittmachers verbindbaren zweiten Elektrode (E₂) zum Übertragen der elektrischen Stimulationsimpulse an das reizbare Herzgewebe,
**dadurch gekennzeichnet, dass**
die Elektrodenleitung eine Hauptleitung und Verzweigung aufweist, wobei die erste Elektrode und mindestens eine weitere Elektrode auf der Hauptleitung angeordnet sind und die zweite oder eine weitere Elektrode auf der Verzweigung angeordnet ist.

2. Elektrodenanordnung nach Anspruch 1, **dadurch gekennzeichnet, dass** die Verzweigung der Elektrodenleitung nach dem Einführen verschiebbar ist.

3. Elektrodenanordnung nach einem der vorangegangenen Ansprüche, **dadurch gekennzeichnet, dass** die Elektroden eine fraktale Oberfläche aufweisen.

4. Elektrodenanordnung nach einem der vorangegangenen Ansprüche, **dadurch gekennzeichnet, dass** das Polaritätsverhältnis einstellbar, insbesondere durch Femeinstellmittel programmierbar, ist.

5. Elektrodenanordnung nach einem der vorangegangenen Ansprüche, **dadurch gekennzeichnet, dass** bei einer Zweikammer-Elektrode im Atrium und im Ventrikel je eine erste und zweite Elektrode (11', 12', 14, 15) vorgesehen sind, die flottierend ausgebildet ist.

6. Elektrodenanordnung nach Anspruch 5, **dadurch gekennzeichnet, dass** das für die Stimulation im Ventrikel bestimmte Ende der Elektrodenleitung (10') Abstandhaltermittel (16) aufweist, welche die zur Stimulation im Ventrikel bestimmte erste und zweite Elektrode (14, 15) im Bereich der Herzspitze zentriert halten.

7. Elektrodenanordnung nach einem der vorangegangenen Ansprüche, **dadurch gekennzeichnet, dass** die weitere Elektrode (E3) als eine außerhalb des Herzens angeordnete Flächenelektrode, insbesondere als Gehäuse des Herzschrittmachers, ausgebildet ist.

8. Elektrodenanordnung nach Anspruch 7, **dadurch gekennzeichnet dass** die Flächenelektrode (E3) als indifferente Elektrode geschaltet ist.

## Claims

1. An electrode arrangement for stimulating the heart by means of an implantable cardiac pacemaker for generating electrical pulses, comprising at least a first electrode (E1) which can be connected to a first output of the cardiac pacemaker and which is arranged on an electrode line which can be fitted intracardially and which is not attached to the wall, and a second electrode (E2) for transmitting the electrical stimulation pulses to the excitable heart tissue, which second electrode is spaced from the first electrode and can be connected to a second output of the cardiac pacemaker,
**characterised in that**
the electrode line has a main line and a branching, wherein the first electrode and at least one further electrode are arranged on the main line and the second or a further electrode is arranged on the branching.

2. An electrode arrangement according to claim 1 **characterised in that** the branching of the electrode line is displaceable after insertion.

3. An electrode arrangement according to one of the preceding claims **characterised in that** the electrodes have a fractal surface.

4. An electrode arrangement according to one of the preceding claims **characterised in that** the polarity ratio is adjustable and in particular programmable by fine adjusting means.

5. An electrode arrangement according to one of the preceding claims **characterised in that** in the case of a dual-chamber electrode provided in the atrium and in the ventricle are first and second respective electrodes (11', 12', 14, 15) which are of a floating nature.

6. An electrode arrangement according to claim 5 **characterised in that** the end of the electrode line (10'), which is intended for stimulation in the ventricle, has spacing means (16) which hold the first and second electrodes (14, 15) intended for stimulation in the ventricle in centred relationship in the region of the tip of the heart.

7. An electrode arrangement according to one of the preceding claims **characterised in that** the further electrode (E3) is in the form of a surface electrode arranged outside the heart, in particular in the form of the housing of the cardiac pacemaker.

8. An electrode arrangement according to claim 7 **characterised in that** the surface electrode (E3) is connected as an indifferent electrode.

## Revendications

1. Dispositif à électrodes pour la stimulation du coeur à l'aide d'un stimulateur cardiaque implantable pour la production d'impulsions électriques, comportant au moins une première électrode (E1) qui peut être reliée à une première sortie du stimulateur cardiaque et est montée dans un conducteur d'électrode pouvant être placé à l'intérieur du coeur et non fixée à demeure à la paroi, et une seconde électrode (E2) distante de la première électrode et pouvant être reliée à une seconde sortie du stimulateur cardiaque, pour la transmission des impulsions électriques de stimulation au tissu cardiaque excitable,
**caractérisé en ce que**
le conducteur d'électrode comporte un conducteur principal et une ramification, la première électrode et au moins une autre électrode étant disposées dans le conducteur principal, et la seconde électrode ou une autre électrode étant disposée dans la ramification.

2. Dispositif à électrodes selon la revendication 1, **caractérisé en ce que** la ramification du conducteur à électrode peut être déplacée après l'introduction.

3. Dispositif à électrodes selon l'une des revendications précédentes, **caractérisé en ce que** les électrodes possèdent une surface fractale.

4. Dispositif à électrodes selon l'une des revendications précédentes, **caractérisé en ce que** le rapport de polarité est réglable, et notamment est programmable à l'aide de moyens de réglage à distance.

5. Dispositif à électrodes selon l'une des revendications précédentes, **caractérisé en ce que** dans le cas d'un fonctionnement à deux chambres, il est prévu dans l'oreillette et dans le ventricule respectivement une première électrode et une seconde électrode (11', 12', 14, 15), qui sont disposées de manière à être flottantes.

6. Dispositif à électrodes selon la revendication 5, **caractérisé en ce que** l'extrémité prévue pour la stimulation dans le ventricule, du conducteur d'électrode (10) comporte des moyens d'entretoisement (16), qui maintiennent à l'état centré les première et seconde électrodes (14, 15), destinées à réaliser la stimulation dans le ventricule, au niveau de la pointe du coeur.

7. Dispositif à électrodes selon l'une des revendications précédentes, **caractérisé en ce que** l'autre électrode (E3) est agencée sous la forme d'une électrode d'une certaine surface, disposée à l'extérieur du coeur, notamment sous la forme d'un boîtier du stimulateur cardiaque.

8. Dispositif à électrodes selon la revendication 7, **caractérisé en ce que** l'électrode d'une certaine surface (E3) est branchée en tant qu'électrode indifférente.
